# EUROPEAN PATENT SPECIFICATION

(11) **EP 0 838 208 B1**
(45) Date of publication and mention of the grant of the patent: **07.04.2004**
(21) Application number: 96901583.3
(22) Date of filing: 19.01.1996
(51) Int. Cl.: A61H 39/00, A61N 1/16, A61B 5/04

(54) **DEVICE FOR TREATING AN ORGANISM WITH ENERGY**
VORRICHTUNG ZUR BEHANDLUNG EINES ORGANISMUS MIT ENERGIE
DISPOSITIF DE TRAITEMENT ENERGETIQUE D'UN ORGANISME

(30) Priority: 11.07.1995 RU 95111981
(43) Date of publication of application: 29.04.1998
(73) Proprietor: Denisov, Stanislav Georgievich, Moscow, 103536 (RU); Ataev, Dzhavanshir Ismail Ogly, Moscow 115569 (RU); Neiman, Viktor Georgievich, Moscow, 127591 (RU); Okhatrin, Anatoly Fedorovich, Moscow, 125299 (RU)
(72) Inventor: Denisov, Stanislav Georgievich, Moscow, 103536 (RU); Ataev, Dzhavanshir Ismail Ogly, Moscow 115569 (RU); Neiman, Viktor Georgievich, Moscow, 127591 (RU); Okhatrin, Anatoly Fedorovich, Moscow, 125299 (RU)
(74) Representative: Kruspig, Volkmar, Dipl.-Ing.
(86) International application number: PCT/RU1996/000014
(87) International publication number: WO 1997/002800

(56) References cited:
- EP-A- 0 229 874
- EP-A- 0 259 769
- DE-A- 3 900 652
- FR-A- 2 236 521
- FR-A- 2 576 515
- RU-C- 2 011 373
- SU-A- 1 602 452

## Description

The invention concerns to the area of a medical engineering and is intended for power effect to bio-object to protect its from harmful radiation of a various nature and reduction in equilibrum condition of its own energo-information field. Magnetic, electrical, electro-magneting fields of chemical substances, radio-active elements and other fields are varied in time, can be referred to harmful radiation fields.

From the document DE-A-3304155 there is known a device for neutralization earthly radiation, containing combination of several coils, located on the general basis consistently one by one at some distance. This device provides partial suppression natural and artificial radiation with electro-magnetic components.

DE-A-3416157 there is known a device for elimination of effect radiation on the ground surface which contain wire to be twisted as spiral winding and bent on open-ended curve. This device acts as a receptive aerial and it is intended for accommodation along the surface of the ground in residential and industrial premises.

DE-A-3515307 there is known a device for shielding or neutralisation from undesirable effects of radiation or fields to bio-object, containing the ring element which is formed by several coils of open-ended wire, on which additional open-ended winds are located. This device neutralizate undesirable radiation or fields by atmospheric and/or technical origin, however does not provide total neutralisatino against harmful fields in all range of frequencies.

FR-A-2534142 there is known a device of constant protection of live substances, especially the person, against dangerous radiation, is containing the set of solenoids and adjustable capacities. The indicated device has primary use only in roentgenologya.

DE-A-3541480 there is known also a device of the energetics effect to bi-object, accepted as prototip, in which the effect is appears by creation of the energetics field, in which the parameters are determined by a chosen geometry of the form for main elements. Indicated device contains the conductor, it is fixed on a substrate as an open-ended space spiral curve with not intersecting coils. Despite of positive influence as a whole, the device does not permit to create the energetics field of sufficient efficiency and does not provide opportunity of regulation its parameters.

There is known the way to appraise efficiency of affect, which concluded to define the organizm's initial condition by Riodorac's method i.e. there are compare results of measurements befor and after influence is (Collection "Medic-technical aspects of reflex-diagnostic and reflex-therapy", 1990, Harkov, page 19-21). However, informable of this way is not large.

There is known the way to appraise efficiency of work by energetic effect device to a bio-object which is occur with the help various medical factores, which are realized with help of device.This way consist of previously measure conductivity biologic-active points by Foll's method at ten main points on each foot and brush and then define for each of them initial significance the deviation from given (the given significance is accepted 50+2 scale points of the device), then repeatedly measure conductivity after effect in those points and define the deviation from given significance. Then it is compare both deviations for each of points and on their difference is evaluate the efficiency of work by energetic effect device (N.L.Lupichev, "Electropointing diagnostics, gomeo-therapy and phenomenon faraction", 1990, Moscow, page 5-12). This way is accepted as prototype. Shortcoming of the indicated way is that particular criterion the valuation efficiency the device's work by energetics affect to bio-object is not entered. In each particular case the operator is based at installed by the Foll's technique to tolerance range (it is determined as 50-60 scale points of the device).

The purpose of the invention is to increase of efficiency the affect to a bio-object to have a possibility of regulation the affect parameters and to have the continuous control of chosen parameters, set by the device.

For it the device by energetics affect to bio-object (it contain wire which is fixed on a substrate as an open-ended space spiraling curve with not crossing coils), has the wire represent as the space spiraling-curve coils, thus the device is supplied by the element for regulation intensity of affect. This element executed as closed electro-condactivity contour, which is located with possibility to change its position concerning space spiral-graphic curve. Space spiral-graphic curve can have a ellipsis-graphic form. The wire can be executed as electro-conductiving cover, which is make up dielectric material, from which is executed space spiral-graphic curve. The closed contour can have curve form, located with opportunity of turn and linear moving concerning space spiral-graphic curve. For activization and the increases of efficiency the energetics field of bio-object (which is under influenced), there are located chemical elements in a zone of wire arrangement, there are possession bioenergetics activity elements as tellur, lantan, gadoliney, vismut and etc. To value at efficiency work of device by energetics affect to bio-object is necessary to measure conductivity of biologic-active points by Foll's method at ten main points on each foot and brush before and after affect; to determine for each points its deviation from given significance (the given significance is accepted 50+2 scale points of the device); for each points to determine difference between deviations which was received as result of measurements before and after affect, about size of which can be judge about the efficiency regarding impact, the measurements a conductivity make after period of time, equal 1, 24 or 48 hours, was up, thus efficiency by affect is evaluate at size of deviations conductivity, is received by results its measurement after effect, don't exceeding 40% from size of deviations conductivity, received by results of its measurement befor the affect. At that it should take into account, that the indications conductivity for bodies with chronic illnesses tend to approximate the significance initial deviation of conductivity during a time, as rule, don't be exceeding 48 hours.

Essence of the invention is explains by the drawing, where is shown a general sight of the device from above in Fig.1, Fig.2 shows variant of fulfillment space spiral-graphic curve in a ellips form, Fig.3 shows fragment of the wire.

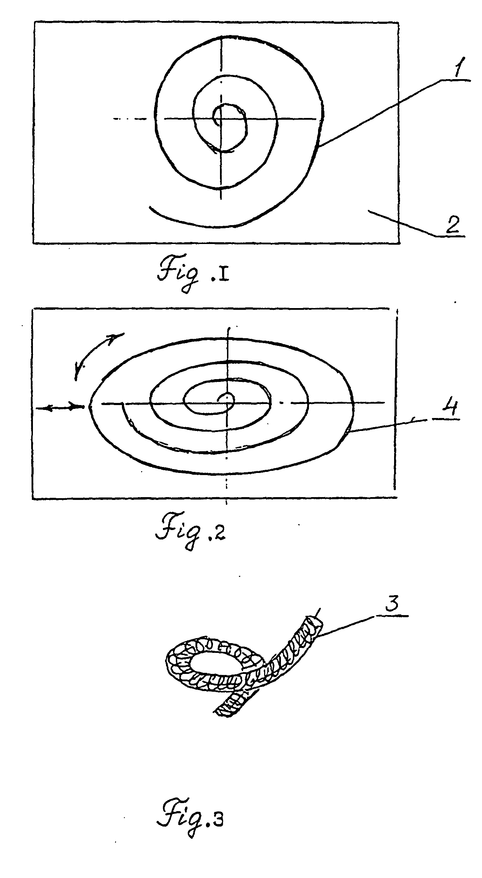

The device contains the wire (1), is executed as space spiralgraphic curve with not crossing coils. The wire (1) is fixed on substrate (2). The wire, was performance by coils space spiral-graphic curve, is located in a spiral form (3). The element (4) for regulation the intensity of affect is executed as closed electro-conductive contour, in particular, as curve form, and is located with opportunity of turn and linear moving relatively the wire (in Fig.2 it is shown by arrows). In situation, shown in Fig.2, element (4) can stop completely the device's functioning and to reduce its affecting intensity to a zero. The wire can be executed as electro-conductive cover, which is puting on fielectric material, for example, as strap. The having bio-energetic activity chemical elements (is not shown in drawing) can be located on substrate (2) in zone of wire arrangement. As such elements there are can be chosen tellur, lantan, gadoliney, vismut and etc.

The device are using thus way. It is put up by substrate on bioobject, in particular on the person body, to a place, indicated by doctor in dependence was diagnose. At using the device it is not required to take off clothes, but distance to body should not exceed 30 cm.

The device, not containing bio-active chemical elements, conditionally named Neutralizator, can be effectively used at treatment illnesses, connected with inflammatory processes in tissue, with increased of body or separate bodies temperature, with sharp pain and etc.. In this cases the device is established on the place of pain's display. A time to carry into effect of session, quantity of sessions, intervals between them, duration the rate of treatment, and also their periodicity are defined by the doctor with view of efficiency of device's effect.

The device, containing located in zone of wire arrangement having by bio-energetics activity chemical elements, conditionally named the Activator, can be effectively used at illnesses, which are stipulated by downturn vital tones, at lowered temperature of body or separate bodies, at decrease of energetics level the own field bio-object (person). Device - activator is established on the place of pain's display. It is possible device establishing on clothes or at a some distance, but not exceeding 20 cm. The time of session effect, quantity of sessions, duration of treatment rate and so on, are selected also by the doctor in view of efficiency the device's effect.

Both the device - Neutralizator and the device - Activator can be used to eliminate of influence harmful external radiation to bioobject, which (radiation) is formed by radiation of household and industrial equipment, by geo-photogen zones and other adverse factors, which are determined by existence in environmental space of radiation at very wide frequent range. For this the element to be in regulation of intensity, must be displace from substrate not less than 1 m. In this case the device can be used at mode of continious work. At that the protective field is formed at radius one or two meters around of the device and all environmental bio-objects are protected against harmful radiation of factors are listed above. This promotes restoration of harmony energo-informatics the bio-objec's field with the environmental world, it has wholesome effect to energetic resources of organism, preventing occurrence functional and then organic illnesses, and even eliminating them.

The valuation efficiency of the device's work by energetics effect to bio-object is executed as follows.

There are previously measurements of conductivity biologic-active points by Foll's method at patient, using for this ten main points on each foot and brushes and define for each of them primary deviation from given significance, which accept 50+2 scale points of the device. Make impact, after which repeatedly measure conductivity in those points later 1, 24, 48 hours after affect. These data can be record in the table (see appendix). Define deviation of each measured significance conductivity from given for all points. Evaluate them in percentage with comparison primary deviation before affect for each appropriate point. Think duration and intensity by affect sufficient, if the significance of deviation after affect does not exceed 40% from size of deviation conductivity, received as results of its measurements up to affect.

The invention permits with sufficient degree authenticity to evaluate influence to bio-object of influencing energetics factors, generated by device, that promotes execution the healthfull and preventive actions with minimum costs.

Device permits to neutralize harmful radiation by various nature, promoting preservation energo-informatics system of bio-object, also to maintenance it at optimum condition, activization and restoration of exhausted energetics resources of organism.

**Table No. 1**

| F.I.O. R.M.G. | | | | | | | | | |
|---|---|---|---|---|---|---|---|---|---|
| Age: 28 years | | | | | | | | | |
| Diagnosis: Scar of duodenum | | | | | | | | | |
| Medicinal instrument: Activator | | | | | | | | | |
| No. P/P | MER. | D | | | | S | | | |
| | | K | 1h | 24h | 48h | K | 1h | 24h | 48h |
| I | Li | 48 | 5I | 52 | 52 | 50 | 55 | 52 | 53 |
| 2 | p | 44 | 48 | 5C | 5I | 43 | 50 | 5I | 5I |
| 3 | Gi | 50 | 5I | 5I | 5I | 44 | 50 | 5I | 52 |
| 4 | ND | 38 | 50 | 53 | 52 | 58 | 5I | 52 | 53 |
| 5 | Mc | 42 | 5I | 53 | 52 | 55 | 53 | 52 | 53 |
| 6 | Al | 55 | 54 | 55 | 53 | 53 | 54 | 54 | 55 |
| 7 | PAD | 50 | 5I | 52 | 53 | 52 | 52 | 53 | 54 |
| 8 | TR | 48 | 50 | 52 | 52 | 52 | 5I | 52 | 52 |
| 9 | C | 45 | 5I | 53 | 54 | 52 | 52 | 53 | 54 |
| I0 | iG | 23 | 50 | 48 | 48 | 48 | 5I | 52 | 5I |
| II | RP | 5I | 53 | 55 | 54 | 54 | 53 | 5I | 52 |
| 12 | F | 60 | 55 | 54 | 56 | 55 | 54 | 55 | 55 |
| I3 | SD | 43 | 5I | 52 | 53 | 42 | 52 | 53 | 53 |
| I4 | E | 76 | 55 | 54 | 53 | 62 | 60 | 55 | 53 |
| 15 | STD | 48 | 5I | 5I | 52 | 52 | 53 | 5I | 5I |
| I6 | K | 55 | 56 | 53 | 54 | 53 | 54 | 55 | 52 |
| I7 | GD | 50 | 52 | 53 | 54 | 5I | 52 | 52 | 54 |
| I8 | VB | 63 | 58 | 56 | 55 | 48 | 56 | 55 | 56 |
| I9 | R | 55 | 54 | 55 | 55 | 54 | 56 | 55 | 55 |
| 20 | V | 52 | 53 | 54 | 55 | 5I | 53 | 54 | 55 |

**Table No. 2**

| F.I.O. SCHTSCH. N.A. | | | | | | | | | |
|---|---|---|---|---|---|---|---|---|---|
| Age: 30 years | | | | | | | | | |
| Diagnosis: Osteochondropathia, radiculit | | | | | | | | | |
| Medicinal instrument: Neutralizer, activator | | | | | | | | | |
| No. P/P | HER. | D | | | | S | | | |
| | | K | 1h | 24h | 48h | K | 1h | 24h | 48h |
| I | Li | 52 | 53 | 54 | 53 | 52 | 52 | 53 | 54 |
| 2 | P | 5I | 52 | 5I | 52 | 52 | 52 | 53 | 53 |
| 3 | Gi | 48 | 50 | 5I | 53 | 44 | 5I | 52 | 53 |
| 4 | ND | 38 | 50 | 5I | 52 | 36 | 50 | 50 | 48 |
| 5 | MC | 52 | 53 | 54 | 55 | 55 | 53 | 54 | 55 |
| 6 | Al | 50 | 50 | 5I | 5I | 48 | 50 | 5I | 5I |
| 7 | PAD | 46 | 49 | 5I | 47 | 48 | 50 | 5I | 50 |
| 8 | TR | 50 | 50 | 50 | 5I | 48 | 50 | 5I | 5I |
| 9 | C | 42 | 50 | 5I | 50 | 52 | 52 | 5I | 53 |
| I0 | iG | 43 | 50 | 5I | 52 | 50 | 5I | 52 | 52 |
| II | RP | 55 | 57 | 56 | 55 | 56 | 57 | 55 | 56 |
| I2 | F | 53 | 56 | 53 | 52 | 55 | 55 | 54 | 52 |
| I3 | SD | 28 | 48 | 5I | 52 | 22 | 50 | 52 | 52 |
| I4 | E | 50 | 53 | 53 | 55 | 55 | 53 | 53 | 53 |
| I5 | STD | 53 | 53 | 58 | 56 | 54 | 53 | 57 | 57 |
| I6 | K | 60 | 54 | 55 | 54 | 6I | 52 | 53 | 53 |
| I7 | GD | 55 | 54 | 55 | 54 | 56 | 54 | 54 | 55 |
| I8 | VB | 50 | 5I | 5I | 5I | 48 | 50 | 5I | 5I |
| I9 | K | 48 | 50 | 5I | 52 | 46 | 50 | 52 | 5I |
| 20 | V | 40 | 50 | 5I | 53 | 38 | 5I | 52 | 53 |

**Table No. 3**

| F.I.O. L.N.A. | | | | | | | | | |
|---|---|---|---|---|---|---|---|---|---|
| Age: 28 years | | | | | | | | | |
| Diagnosis: NCD hypertens. types | | | | | | | | | |
| Medicinal instrument: Neutralizer | | | | | | | | | |
| No. P/P | MER. | D | | | | S | | | |
| | | K | 1h | 24h | 48h | K | 1h | 24h | 48h |
| I | Li | 44 | 5I | 52 | 52 | 45 | 5I | 52 | 52 |
| 2 | P | 52 | 5I | 52 | 52 | 52 | 5I | 52 | 52 |
| 3 | Gi | 50 | 5I | 52 | 52 | 46 | 5I | 52 | 52 |
| 4 | ND | 44 | 50 | 5I | 5I | 43 | 49 | 5I | 5I |
| 5 | MC | 32 | 50 | 5I | 52 | 45 | 5I | 5I | 5I |
| 6 | A1 | 48 | 50 | 5I | 5I | 47 | 5I | 5I | 5I |
| 7 | PAD | 5I | 52 | 53 | 53 | 5I | 52 | 53 | 53 |
| 8 | TR | 46 | 50 | 52 | 52 | 45 | 5I | 52 | 52 |
| 9 | C | 50 | 50 | 5I | 52 | 42 | 5I | 5I | 52 |
| I0 | iG | 44 | 50 | 5I | 5I | 43 | 52 | 5I | 5I |
| II | RP | 55 | 53 | 54 | 54 | 55 | 53 | 53 | 54 |
| I2 | F | 54 | 54 | 53 | 54 | 54 | 54 | 53 | 53 |
| I3 | SD | 43 | 50 | 53 | 53 | 42 | 5I | 52 | 53 |
| I4 | E | 58 | 52 | 53 | 53 | 60 | 5I | 52 | 52 |
| I5 | STD | 52 | 53 | 52 | 52 | 52 | 53 | 53 | 52 |
| I6 | K | 5I | 52 | 53 | 53 | 5I | 52 | 53 | 53 |
| I7 | GD | 52 | 52 | 53 | 52 | 5I | 52 | 53 | 53 |
| I8 | VB | 48 | 50 | 53 | 52 | 43 | 52 | 53 | 53 |
| 19 | R | 56 | 53 | 54 | 55 | 58 | 52 | 55 | 54 |
| 20 | V | 50 | 50 | 52 | 52 | 48 | 5I | 52 | 53 |

**Table No. 4**

| F.I.O. K.O.A. | | | | | | | | | |
|---|---|---|---|---|---|---|---|---|---|
| Age: 78 years | | | | | | | | | |
| Diagnosis: Arthropathy, podagra | | | | | | | | | |
| Medicinal instrument: Activator | | | | | | | | | |
| No. P/P | MER. | D | | | | S | | | |
| | | K | 1h | 24h | 48h | K | 1h | 24h | 48h |
| I | Li | 43 | 50 | 50 | 5I | 42 | 5I | 50 | 5I |
| 2 | P | 50 | 5I | 52 | 53 | 5I | 53 | 52 | 52 |
| 3 | Gi | 48 | 50 | 5I | 52 | 40 | 50 | 52 | 52 |
| 4 | ND | 30 | 50 | 48 | 50 | 28 | 48 | 50 | 5I |
| 5 | MC | 30 | 5I | 5I | 5I | 44 | 50 | 5I | 5I |
| 6 | Al | 48 | 5I | 5I | 50 | 50 | 50 | 5I | 50 |
| 7 | PAD | 50 | 5I | 50 | 5I | 5I | 5I | 50 | 50 |
| 8 | TR | 48 | 50 | 50 | 50 | 50 | 5I | 50 | 5I |
| 9 | C | 48 | 50 | 5I | 53 | 43 | 5I | 53 | 53 |
| I0 | iG | 43 | 50 | 5I | 50 | 48 | 5C | 50 | 50 |
| II | RP | 48 | 50 | 5I | 5I | 49 | 50 | 5I | 5I |
| I2 | F | 50 | 5I | 52 | 53 | 53 | 53 | 53 | 53 |
| I3 | SD | 30 | 50 | 45 | 40 | 28 | 5I | 46 | 42 |
| I4 | E | 50 | 5I | 52 | 5C | 44 | 50 | 52 | 50 |
| I5 | STD | 44 | 48 | 50 | 5I | 43 | 50 | 5I | 52 |
| I6 | K | 33 | 50 | 5I | 30 | 38 | 49 | 5I | 50 |
| I7 | GD | 48 | 50 | 5I | 50 | 46 | 5I | 50 | 5I |
| I8 | V3 | 47 | 50 | 5I | 50 | 46 | 5I | 52 | 50 |
| I9 | K | 32 | 46 | 50 | 5I | 28 | 48 | 5I | 50 |
| 20 | V | 26 | 48 | 45 | 44 | 27 | 47 | 48 | 50 |

## Claims

1. Device for influencing an organism using environmental energy, wherein the device has a wire (1), mounted on a substrate (2) in an open-ended spiral form spaced apart with non-intersecting windings, the wire (1) is coiled in a winding helical form spaced apart
**characterised in that** the device is provided with a regulating element (4) to influence the intensity of the energy and said element (4) is executed as a closed electrically conductive contour situated substantially in the same plane as the wire (1), which contour is arranged with the possibility to change its position in respect to the wire (1) winding.

2. Device according to claim 1,
**characterised in that**
said wire (1) winding has an elliptical form.

3. Device according to claim 1 or 2,
**characterised in that**
said wire (1) is executed with an electrically conductive coating, encased by a dielectrical material.

4. Device according to claims 1, 2, or 3,
**characterised in that**
the closed contour has the shape of a curve which is arranged with the possibility to turn and to move linearly in respect to the winding.

5. Device according to the preceding claims 1 to 4,
**characterised in that**
the device has chemical elements having a bio-energetic activity and arranged in a region of the wire winding.

6. Device according to claim 5,
**characterised in that**
tellurium, lanthanum, gadolinium, bismuth, etc. are used as qualitative bioactive chemical elements.

## Patentansprüche

1. Vorrichtung zum Beeinflussen eines Organismus unter Verwendung umgebender Energie, wobei die Vorrichtung einen Draht (1) aufweist, der an einem Substrat (2) in offenendiger, beabstandeter Spiralform mit sich nicht überschneidenden Wicklungen angebracht ist,
**dadurch gekennzeichnet, dass**
der Draht (1) in beabstandeter Schraubenzylinderform gewickelt ist, wobei die Vorrichtung mit einem Regulierungselement (4) zur Beeinflussung der Intensität der Energie versehen ist und dieses Element als eine geschlossene, elektrisch leitfähige Außenlinie ausgeführt ist, die im wesentlichen in derselben Ebene wie der Draht (1) angeordnet ist, wobei die Außenlinie mit der Möglichkeit, ihre Position in bezug auf die Drahtwicklung zu verändern, angeordnet ist.

2. Vorrichtung nach Anspruch 1,
**dadurch gekennzeichnet, dass**
die Drahtwicklung (1) eine elliptische Form hat.

3. Vorrichtung nach Anspruch 1 oder 2,
**dadurch gekennzeichnet, dass**
der Draht (1) als elektrisch leitfähiger Überzug ausgeführt ist, der ein dielektrisches Material umgibt.

4. Vorrichtung nach einem der Ansprüchen 1, 2 oder 3,
**dadurch gekennzeichnet, dass**
die geschlossene Außenlinie die Form einer Biegung hat, die mit einer Möglichkeit zum Drehen und geradlinig Bewegen in bezug auf die Wicklung angeordnet ist.

5. Vorrichtung nach den vorhergehenden Ansprüchen 1 bis 4,
**dadurch gekennzeichnet, dass**
sie chemische Elemente aufweist, die eine bioenergetische Aktivität haben und die in einem Bereich der Drahtwicklung angeordnet sind.

6. Vorrichtung nach Anspruch 5,
**dadurch gekennzeichnet, dass**
als qualitative bioaktive chemische Elemente Tellur, Lanthan, Gadolinium, Wismut und so weiter verwendet werden.

## Revendications

1. Dispositif pour influencer un organisme en utilisant l'énergie environnementale, ledit dispositif comprenant un fil (1) monté sur un substrat (2) sous la forme d'une spirale à extrémités ouvertes et écartée avec des enroulements qui ne se recoupent pas,
**caractérisé en ce que**
le fil (1) est rubané sous forme d'enroulements hélicoïdaux écartés, dans lequel le dispositif est pourvu d'un élément de régulation (4) pour influencer une intensité de l'énergie, et ledit élément (4) est exécuté sous la forme d'un contour électriquement conducteur fermé situé sensiblement dans le même plan que le fil (1), et ledit contour est agencé avec possibilité de changer sa position par rapport à l'enroulement de film (1).

2. Dispositif selon la revendication 1, **caractérisé en ce que** ledit enroulement de fil (1) a une forme elliptique.

3. Dispositif selon l'une ou l'autre des revendications 1 et 2, **caractérisé en ce que** ledit fil (1) est exécuté avec un revêtement électriquement conducteur, enchâssé par un matériau diélectrique.

4. Dispositif selon l'une des revendications 1, 2 et 3, **caractérisé en ce que** le contour fermé a la forme d'une courbe qui est agencée avec possibilité de tourner et de se déplacer linéairement par rapport à l'enroulement.

5. Dispositif selon l'une des revendications précédentes 1 à 4,
**caractérisé en ce que** le dispositif comprend des éléments chimiques ayant une activité bio-énergétique et agencés dans une région de l'enroulement de fil.

6. Dispositif selon la revendication 5, **caractérisé en ce que** l'on utilise à titre d'éléments chimiques bio-actifs du tellure, du lanthane, du gadolinium, du bismuth, etc.
